# EUROPEAN PATENT SPECIFICATION

(11) **EP 1 618 924 B1**
(45) Date of publication and mention of the grant of the patent: **09.09.2009**
(21) Application number: 04077104.0
(22) Date of filing: 20.07.2004
(51) Int. Cl.: A61N 5/10

(54) **Device for radiation treatment of proliferative tissue surrounding a cavity in an animal body**
Einrichtung zur Strahlenbehandlung von proliferativem Gewebe grenzend an eine Körperhöhle
Dispositif d'irradiation de tissu proliférant entourant une cavité corporelle

(43) Date of publication of application: 25.01.2006
(73) Proprietor: Nucletron B.V., 3905 TH Veenendaal (NL)
(72) Inventor: Kindlein, Johann, 47918 Toenisvorst (DE); Koster, Albert Dirk Adrianus, 3523 EL Utrecht (NL)
(74) Representative: Valkonet, Rutger

(56) References cited:
- EP-A- 1 402 922
- US-A- 3 872 856
- US-A- 5 618 266
- US-A- 5 913 813
- US-A- 5 938 582
- US-B1- 6 390 968
- US-B1- 6 572 633

## Description

The invention relates to a device for radiation treatment of proliferative tissue surrounding a cavity in an animal body comprising:
- at least a first inflatable balloon system having a balloon wall for placement in said cavity;
- a supportive probe having an elongated, hollow body with a distal end connected with said at least first inflatable balloon system and a proximal end remaining outside said cavity;
- inflation means for inflating and deflating said at least first balloon system;
- radiation delivering means for placing at least one energy emitting source within said cavity for performing said radiation treatment, wherein said radiation delivering means comprise at least one hollow guidance channel for guiding said at least one energy emitting source inside said cavity.

Such device is for example known from European patent application no. 1 402 922 A1 in the name of the present applicant, or in the U.S. Patent US 6,572,633.

In this patent publication an apparatus is described for treating tissue surrounding a surgically excised tumour with radioactive emissions to kill any cancer cells that may be present in the margins surrounding the excised tumour. Following surgical removal of a tumour, say in the brain or breast, a supportive probe having an inflatable chamber connected at a distal end thereof is introduced into the removal of a tumour. Subsequently the deflated chamber is inflated by suitable inflation means by injecting a fluid within the distensible reservoir formed by the chamber, via a passageway in the supportive probe.

The balloon chamber will take up a fully spherical geometrical shape after inflation. As the cavities created by surgery in the body to be treated normally do not exhibit a perfect spherical cavity at some locations across the circumferential surface of the inflated balloon chamber pressure points may occur due to the high forces exerted by the balloon wall on the tissue surrounding the cavity.

The high forces exerted by the inflated balloon chamber result in tissue perfusion problems, like pressure-induced damage like necrosis or tissue infarction.

The invention aims to provide a device for radiation treatment of proliferative tissue surrounding a cavity in an animal body according to the above identified preamble, which allows the insertion of an inflatable balloon system into a body cavity created due to the surgical removal of a cancer tumour, which balloon system completely conforms the outer surface of the cavity disregarding its outer shape or dimensions and which avoids the risk of tissue perfusion as no high pressures are exerted by the balloon system on the tissue surrounding the cavity.

The device is according to the invention **characterized in that** said inflatable balloon system comprises two or more balloon chambers accommodated around the distal end of said supportive probe, which when inflated are capable of completely conforming the inner surface of the cavity and wherein said at least one guidance channel is positioned between and outside adjacent balloon chambers on the outer circumferential surface of the supportive probe. With this feature an inflated balloon system is obtained with a shape conformal to the body cavity shape. As the balloon system does not necessarily exhibit a spherical shape when inflated, any pressure-induced damage like necrosis or tissue infarction due to high forces exerted by spherically inflated balloon systems as with the known balloon applicators is avoided.

The balloon applicator according to the invention can now advantageously also be used in body cavities, which do not exhibit a perfect spherical geometry.

More in particular said balloon chambers can be formed by locally connecting said balloon wall to the elongated probe body, thereby forming creating a number of balloon wall folds.

Especially said number of balloon wall folds equals the number of guidance channels.

In a specific embodiment said connections between said balloon wall and said elongated body extend in longitudinal direction of the elongated probe body and more in particular said connections are oriented parallel to the orientation of the guidance channels on said elongated probe body a conformal dose distribution of the several energy emitting sources inserted at different locations within the several guidance channels can be obtained, resulting in more accurate radiation treatment sessions.

In two embodiments said connections between said balloon wall and said elongated probe body are established by means of glueing or by means of welding.

More in particular said balloon system exhibits a balloon wall surface being greater than the proliferative tissue surface surrounding said cavity such that the balloon system may have a shape conformal to the body cavity shape.

By manufacturing said inflatable balloon system from a flexible foil, e.g. polyurethane (PUR) a balloon system is obtained with excellent tear, cutting and puncture characteristics thereby obviating the risk of damage or malfunction. PUR is an elastic and extremely thin material that returns to its original shape without any relevant memory.

Moreover, when pressurized the thin PUR balloon system can be shaped in complex balloon shapes and it does tend to a spherical shape.

Also other polymer materials with similar characteristics can be used.

In another advantageous embodiment a radiation shielding material is positioned inside said hollow, elongated probe body and at least partly covering the inner surface of said hollow, elongated probe body. Herewith the propagation of radiation emitted by the energy emitting sources inserted in the guidance channels through the supportive probe is obviated and radiation is emitted only in a radial direction away from the supportive probe towards the proliferative tissue surrounding the cavity. This results in a more accurate radiation dose distribution, which distribution is more conformal with the desired dose distribution as pre-planned and desired.

In an specific embodiment said radiation shielding material is interrupted by means of a slit extending in longitudinal direction of the elongated probe body, wherein said interruption slit exhibits an n.¼ circle segment with n being 1, 2 or 3.

This embodiment allows radiation to be emitted in a specific direction by a energy emitting source inserted in the supportive probe to propagate through the supportive probe and the balloon system towards the proliferative tissue surrounding the cavity. With the use of interruptions with a specific n.¼ circle segment cross section, also radiation emitted in a longitudinally extending beam with a n.¼ circle segment cross section will be obtained, allowing radiation treatments to be performed under specific conditions and directions.

In other embodiments the energy emitting source can be a High Dose Rate Ir-192 source, or a Pulse Dose Rate Ir-192 source, or a miniature X-ray source. However the invention is not limited to the use of these kinds of energy emitting sources.

More in particular the dose distribution generated with said plurality of energy emitting sources positioned in said plurality of hollow guidance channels will follow the conformal shape of the outside of the balloon wall of the first inflatattable balloon system, which is surrounded by the tissue.

In another specific embodiment the device comprises an open drainage catheter for insertion in the hollow, elongated probe body for draining body liquids out of the cavity after the applied radiation treatment.

The invention will now be described with reference to a drawing, which shows in:
Figure 1 an embodiment of a device according to the state of the art;
Figure 2 a first embodiment of a device according to the invention;
Figures 3a-3c another embodiments of a device according to the invention;
Figure 4 a perspective view of the embodiment of Figure 2;
Figure 5 a detailed view of an embodiment according to the invention.

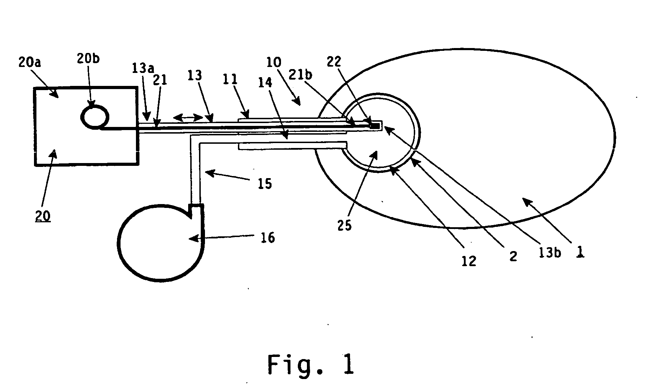

Figure 1 discloses a lateral view of an embodiment of a device for radiation treatment of proliferative tissue surrounding a cavity in an animal body according to the invention. In this drawing a part of the animal body is depicted with reference numeral 1, for example the head of a patient, or a breast of a woman. A cancer tumour has been removed from said part 1 of said animal body during a surgical procedure leaving a cavity 2. As any cancer cell may still be present in the margins surrounding the surgically excised tumour in said cavity 2 a radiation treatment of said cancer cell is desirable with the use of radioactive emissions from energy emitting sources positioned inside said cavity 2.

To this end an applicator 10 is introduced into the cavity 2, which device 10 comprises a supportive probe 11 having an inflatable balloon system 12 connected to a distal end 11a of said supportive probe 11. Once the deflated balloon system 12 has been introduced inside the cavity 2, the balloon system 12 is inflated by suitable inflation means 16 by injecting a pressurized medium 25 (for example a fluid) via a passageway 15 and a passageway 14 in the supportive probe 11 towards the distensible reservoir formed by the balloon system 12.

In addition, the supportive probe 11 is provided with a guidance channel through which a flexible catheter tube 13 is guidable until it extends with its distal end 13b within the cavity 2. The catheter tube 13 is connected with its proximal end 13a with radiation delivery means 20, here a remote afterloader apparatus 20 for performing radiation therapy treatments of the cancer tissue surrounding the cavity 2. The afterloader apparatus 20 contains a radiation shielded compartment 20a, in which compartment an solid energy emitting source 22 is accommodated. The energy emitting source 22 is attached to a distal end 21b of a source wire 21, which source wire can be advanced through the hollow catheter tube 13 by means of wire drive means 20b. The solid energy emitting source 22 can be advanced from said radiation shielded compartment 20a through the hollow catheter tube 13 towards a desired location within the cavity 2.

The positioning of the solid energy emitting source 22 at different locations within its hollow catheter tube 13 and in the cavity 2 gives more possibilities for performing a radiation therapy treatment session. The total dose distribution of the tissue to be treated will be conformal with the volume of the tumour tissue surrounding the cavity 2 by optimizing the dwell times for the different positions within the cavity 2 of the solid energy emitting source 22. Moreover the guidance of the solid energy emitting source 22 through the hollow catheter tube 13 within the cavity 2 allows a temporary insertion of the source 22 in the reproducible manner at different locations.

Often multiple guidance channels suited for several catheter tubes 13 are implemented allowing the insertion of several energy emitting sources 22 at different locations within the cavity, resulting in a more accurate conformal dose distribution.

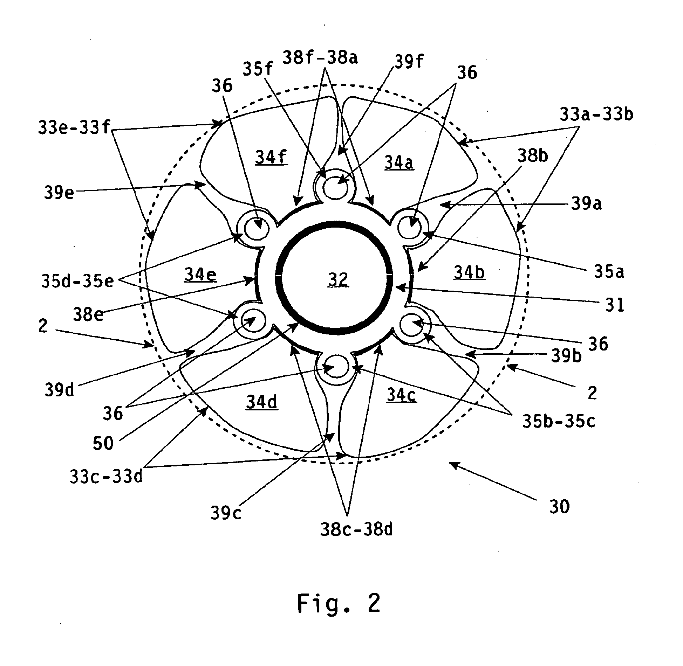

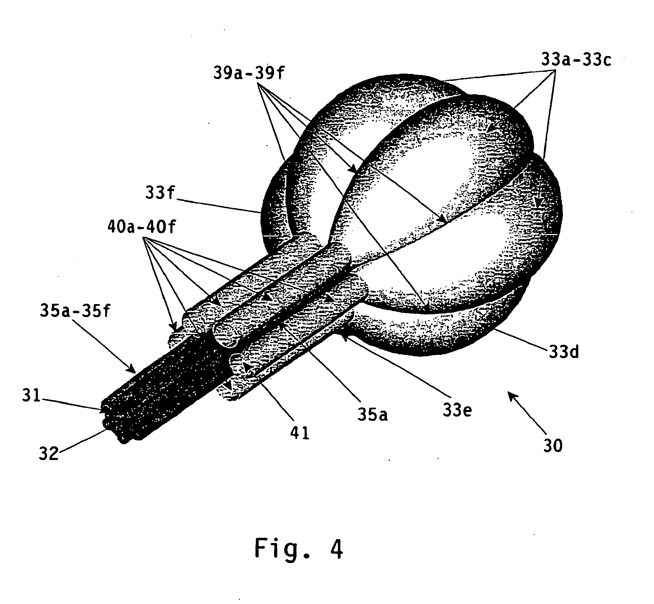

Figure 2 shows a first embodiment of a device according to the invention. Figure 2 depicts the device 30 in a cross sectional view, whereas Figure 4 depicts a complete view of the device 30. The device for radiation treatment of proliferative tissue surrounding a cavity in an animal body consists of a supportive probe 31, which is preferably hollow (reference numeral 32). The balloon system 33 (to be compared with the balloon system 12 of the device according to the prior art as depicted in Figure 1) exhibits a significantly larger outer surface 33a-33f than the inner surface of the cavity 2 within the animal body 1.

With this feature which is to be considered an embodiment of the device according to the invention the outer surface of the balloon system 33 completely conforms the inner surface of the cavity 2 irrespective of the shape or dimensions of the cavity. Hence the construction of the balloon applicator 30 as shown in Figure 2 avoids the risk of tissue perfusion as no pressure points can occur due to possible high forces exerted by the balloon wall on the tissue surrounding the cavity 2. The balloon system 33 does not necessarily exhibit a spherical shape as with the devices according to the state of the art (see Figure 1) when the balloon system 33 is inflated. Hence any pressure-induced damage like necrosis or tissue infarction due to high forces is avoided.

The applicator 30 can advantageously also be used in body cavities 2, which do not exhibit a perfect spherical geometry. As clearly shown in Figure 2 the balloon system 33 comprises several balloon chambers (here six chambers) 34a-34f, which are radially divided around the supportive probe 31. In the embodiment as shown in Figure 2 each balloon chamber 34a-34f is locally attached (reference numerals 38a-38f) to the circumferential surface of the supportive probe 31. Due to the local elongated connections 38a-38f of the several balloon chambers 34a-34f to the elongated supportive probe 31 several balloon wall folds 39a-39f are created between the subsequent adjacent balloon chambers 34a-34f.

The advantage of the use of multiple balloon chambers is that the overall geometry of the inflated balloon system 33 does not necessarily exhibit a spherical balloon shape as with the balloon applicators according to the prior art. Not only pressure points and therefore tissue perfusion problems, like pressure-induced like necrosis and tissue infarction are avoided moreover an uni-conformal dose distribution of the several energy emitting sources introduced into the cavity 2 towards the tissue of the cavity 2 is possible.

Furthermore as the balloon system 33 comprising the several balloon chambers 34a-34f completely conformable to the inner circumference of the cavity 2 no deformation of the cavity and tissue of the cavity will occur but the exact geometry of the cavity being created in said patient's body is maintained. This latter aspect guarantees an accurate emission of radiation towards the tissue surrounding the cavity 2 conformal to the desired radiation treatment session being preplanned prior to the insertion of the balloon applicator 30.

When inflated with higher pressures the balloon system take a well defined shape (spherical, ovoid, etc.) but it will also have the same shape at different diameters, depending on the pressure being applied to the separate balloon chambers.

At a low pressure the balloon system will adopt a shape conformal with the body cavity and can be conformal isodose optimized by using the multiple guiding channels through which the energy emitting sources are inserted and positioned within the cavity.

Hence by implementing the device at different pressures within the balloon system, the device can be used to different types of radiation treatments.

Each connection 38a-38f of the balloon wall 33a-33f with the supportive probe 31 can be performed by means of glueing or welding.

Furthermore the device 30 according to the invention is provided with several guidance channels 35a-35f which are hollow (see reference numeral 36) and which extend in longitudinal direction of the elongated supportive probe 31 parallel and adjacent to the longitudinal connections 38a-38f of the several balloon chambers 34a-34f with the supportive probe 31.

Through the hollow guidance channels 35a-35f several energy emitting sources 22 (see figure 1) can be inserted in a manner as disclosed in Figure 1. This means that in each hollow guidance channel 35a-35b a catheter tube 13 is inserted after which an energy emitting source 22 is introduced into the catheter tube 13 with the use of a source wire 21 and source wire drive means 22b accommodated in an afterloader apparatus 20.

With the use of several guidance channels 35a-35f multiple energy emitting sources 22 can be inserted through corresponding catheter tubes 13 and source wires 21 towards different locations within the cavity 2. Also the dwell times of each energy emitting source 22 at each dwell position within one of said catheter tubes 13 can be chosen dependent on the radiation treatment to be performed and the radiation dose distribution to be exposed towards the cancerous tissue surrounding the cavity 2.

Additionally, the hollow, elongated supportive probe 31 is at its inner surface covered with a radiation shielding material 50. The radiation shielding material 50 avoids the propagation of radiation emitted by the energy emitting sources 22 inserted via catheter tubes 13 in the guidance channels 35a-35f through the supportive probe 31. Hence radiation is emitted only in a radial direction away from the supportive probe 31 towards the proliferative tissue surrounding the cavity 2.

Therefore with the radiation shielding material 50 completely covering the inner surface of the hollow, elongated supportive probe 11 a more accurate radiation dose distribution of the sources 22 towards the tissue of the cavity 2 is obtained, which distribution is more conformal with the desired dose distribution as pre-planned and desired.

In another embodiment of the device according to the invention an energy emitting source can be inserted in the cavity 2 through the hollow, elongated supportive probe 31 in a similar way as with the guidance channels 35a-35f. In that embodiment the radiation shielding material 50 is not completely covering the inner surface of the hollow, elongated supportive probe 31 in order to allow radiation emitted by a source 22' inserted in the supportive probe 31 via a catheter tube 13' to propagate through the supportive probe 31 and the balloon system 12 towards the proliferative tissue surrounding the cavity 2.

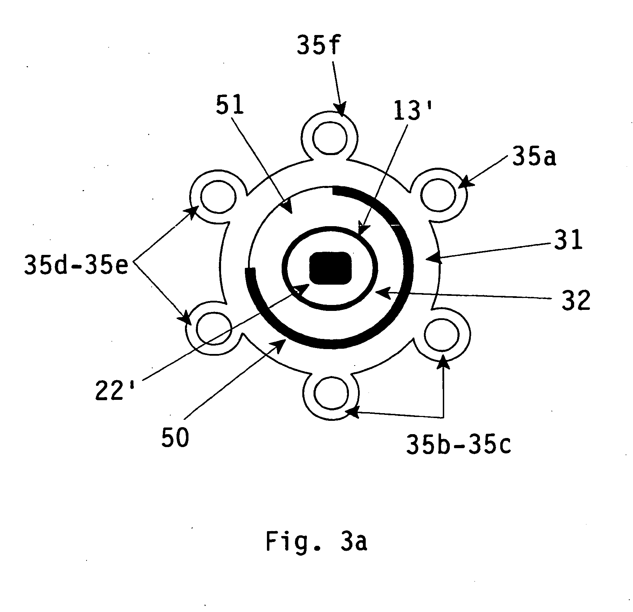

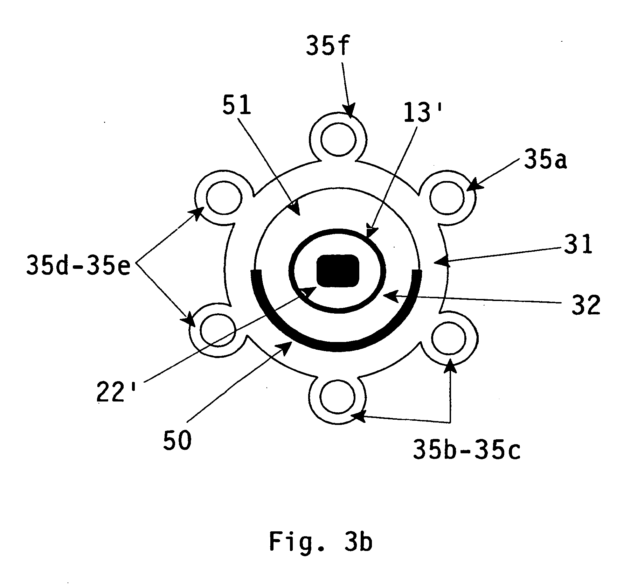

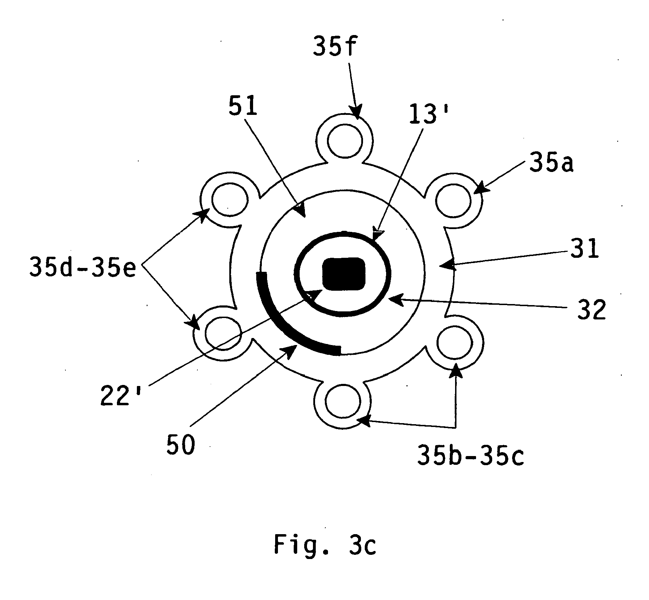

More in particular in that embodiment said radiation shielding material 50 is interrupted by means of a slit 51 extending in longitudinal direction of the elongated probe body 11. See Figure 3a. The interruption slit 51 may exhibit an n.¼ circle segment, with n being 1, 2 or 3 as depicted in Figures 3a-3b-3c. Hence the radiation emitted by the energy emitting source 22' will be emitted in a longitudinally extending beam with a n.¼ circle segment cross section.

Likewise through the hollow, elongated probe body 11 an open drainage catheter (not depicted) can be inserted, which drainage catheter is open at its both ends in order to allow any body liquids to be drained out of the cavity after the applied radiation treatment.

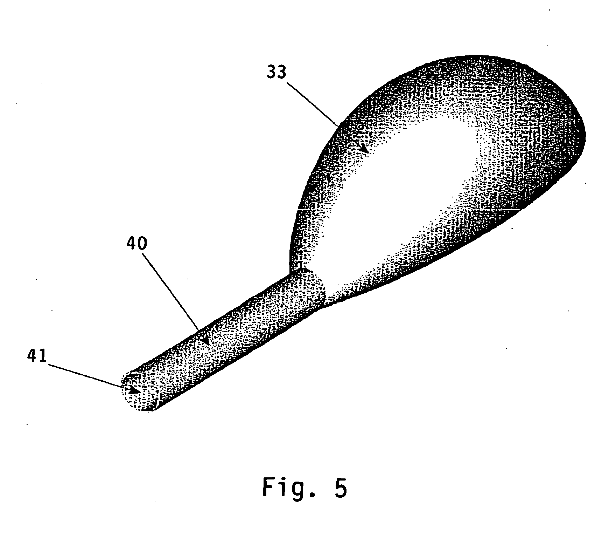

As shown in Figures 4 and 5 the balloon chambers 33a-33f are accommodated around the supportive probe 31. Each balloon chamber 33a-33f is provided with a tube 40a-40f which is hollow (see reference numeral 41) via the hollow supply tubes 40a-40f a medium under pressure can be introduced in each balloon chamber 33a-33f resulting in an inflation of the balloon chambers 33a-33f, which results in an inflation of the balloon chamber.

## Claims

1. Device (30) for radiation treatment of proliferative tissue surrounding a cavity (2) in an animal body (1) comprising:
- at least an inflatable balloon system (33) having a balloon wall for placement in said cavity (2);
- a supportive probe (31) having an outer circumferential surface and having an elongated, hollow body with a distal end connected with said inflatable balloon system and a proximal end remaining outside said cavity (2);
- inflation means for inflating and deflating said balloon system (33);
- radiation delivering means for placing at least one energy emitting source (22) within said cavity (2) for performing said radiation treatment, wherein said radiation delivering means comprise at least one hollow guidance channel for guiding said at least one energy emitting source inside said cavity,
**characterized in that,**
said inflatable balloon system (33) comprises two or more balloon chambers (34a-34f) accommodated around the distal end of said supportive probe (31), which when inflated are capably of completely conforming the inner surface of the cavity (2) and wherein said at least one guidance channel (35a-35f) is positioned between and outside adjacent balloon chambers (34a-34f) on the outer circumferential surface of the supportive probe.

2. Device according to claim 1, **characterized in that**, said balloon chambers are formed by locally connecting (38a-38f) said balloon wall to the elongated probe body (31), thereby forming creating a number of balloon wall folds.

3. Device according to claim 2, **characterized in that**, said number of balloon wall folds equals the number of guidance channels.

4. Device according to claim 2 or 3, **characterized in that**, said connections (38a-38f) between said balloon wall and said elongated body extend in longitudinal direction of the elongated probe body (31).

5. Device according to claim 4, **characterized in that**, said connections are oriented parallel to the orientation of the guidance channels on said elongated probe body.

6. Device according to claim 4 or 5, **characterized in that**, said connections between said balloon wall and said elongated probe body are established by means of glueing.

7. Device according to claim 6 or 7, **characterized in that**, said connections between said balloon wall and said elongated probe body are established by means of welding.

8. Device according to anyone of the preceding claims **characterized in that**, said balloon system exhibits a balloon wall surface being greater than the proliferative tissue surface surrounding said cavity.

9. Device according to anyone of the preceding claims **characterized in that**, said inflatable balloon system is made from a flexible foil, e.g. polyurethane.

10. Device according to anyone of the preceding claims **characterized in that**, a radiation shielding material (50) is positioned inside said hollow, elongated probe body (31) and at least partly covering the inner surface of said hollow, elongated probe body.

11. Device according to claim 10, **characterized in that**, said radiation shielding material (50) is interrupted by means of a slit (51) extending in longitudinal direction of the elongated probe body.

12. Device according to claim 11, **characterized in that**, said interruption slit exhibits an n.% circle segment with n being 1, 2 or 3.

13. Device according to anyone of the claims 1-12, **characterized in that,** the energy emitting source is a High Dose Rate Ir-192 source.

14. Device according to anyone of the claims 1-12, **characterized in that**, the energy emitting source is a Pulse Dose Rate Ir-192 source.

15. Device according claim to anyone of the claims 1-12, **characterized in that**, the energy emitting source is a miniature X-ray source.

16. Device according to anyone of the claims 1-12, **characterized in that**, the energy emitting source is a radio-waves emitting source.

17. Device according to anyone of the preceding claims, **characterized in that**, the dose distribution generated with said plurality of energy emitting sources positioned in said plurality of hollow guidance channels will follow the conformal shape of the outside of the balloon wall of the first inflatattable balloon system, which is surrounded by the tissue.

18. Device according to anyone of the preceding claims, **characterized in that**, the device comprises an open drainage catheter for insertion in the hollow, elongated probe body for draining body liquids out of the cavity after the applied radiation treatment.

## Patentansprüche

1. Vorrichtung (30) zur Strahlentherapie von proliferativem Gewebe, das ein Cavum (2) umgibt, in einem Tierkörper (1), umfassend:
- wenigstens ein aufblasbares Ballonsystem (33) mit einer Ballonwand zur Platzierung in dem Cavum (2);
- eine stützende Sonde (31), die eine äußere Umfangsfläche und einen länglichen Hohlkörper mit einem distalen Ende, das mit dem aufblasbaren Ballonsystem verbunden ist, und einem proximalen Ende, das außerhalb des Cavums (2) bleibt, aufweist;
- ein Inflationsmittel zum Aufblasen und Entleeren des Ballonsystems (33);
- ein Strahlung abgebendes Mittel zum Platzieren wenigstens einer Energie emittierenden Quelle (22) in dem Cavum (2) zur Durchführung der Strahlenbehandlung, wobei das Strahlung abgebende Mittel wenigstens einen hohlen Führungskanal zum Führen der wenigstens einen Energie emittierenden Quelle im Inneren des Cavum umfasst,
**dadurch gekennzeichnet, dass**
das aufblasbare Ballonsystem (33) zwei oder mehr Ballonkammern (34a-34f), angepasst um das distale Ende der stützenden Sonde (31), umfasst, die, wenn sie aufgeblasen werden, fähig sind, sich vollständig an die innere Oberfläche des Cavums (2) anzupassen, und wobei der wenigstens eine Führungskanal (35a-35f) zwischen und außerhalb benachbarter Ballonkammern (34a-34f) an der äußeren Umfangsfläche der stützenden Sonde positioniert ist.

2. Vorrichtung gemäß Anspruch 1, **dadurch gekennzeichnet, dass** die Ballonkammern durch lokales Verbinden (38a-38f) der Ballonwand mit dem länglichen Sondenkörper (31) gebildet sind, wodurch eine Reihe von Ballonwandfalzen geschaffen werden.

3. Vorrichtung gemäß Anspruch 2, **dadurch gekennzeichnet, dass** die Anzahl der Ballonwandfalze gleich der Anzahl der Führungskanäle ist.

4. Vorrichtung gemäß Anspruch 2 oder 3, **dadurch gekennzeichnet, dass** sich die Verbindungen (38a-38f) zwischen der Ballonwand und dem länglichen Körper in Längsrichtung des länglichen Sondenkörpers (31) erstrecken.

5. Vorrichtung gemäß Anspruch 4, **dadurch gekennzeichnet, dass** die Verbindungen parallel zur Orientierung der Führungskanäle an dem länglichen Sondenkörper orientiert sind.

6. Vorrichtung gemäß Anspruch 4 oder 5, **dadurch gekennzeichnet, dass** die Verbindungen zwischen der Ballonwand und dem länglichen Sondenkörper durch Klebung hergestellt sind.

7. Vorrichtung gemäß Anspruch 6 oder 7, **dadurch gekennzeichnet, dass** die Verbindungen zwischen der Ballonwand und dem länglichen Sondenkörper mittels Schweißen hergestellt sind.

8. Vorrichtung gemäß einem der vorangehenden Ansprüche, **dadurch gekennzeichnet, dass** das Ballonsystem eine Ballonwandoberfläche aufweiset, die größer ist als die Oberfläche des poliferativen Gewebes, das das Cavum umgibt.

9. Vorrichtung gemäß einem der vorangehenden Ansprüche, **dadurch gekennzeichnet, dass** das aufblasbare Ballonsystem aus einer flexible Folie, z.B. Polyurethan, hergestellt ist.

10. Vorrichtung gemäß einem der vorangehenden Ansprüche, **dadurch gekennzeichnet, dass** ein gegenüber Strahlung abschirmendes Material (50) im Inneren des hohlen, länglichen Sondenkörpers (31) positioniert ist und wenigstens teilweise die innere Oberfläche des hohlen, länglichen Sondenkörpers bedeckt.

11. Vorrichtung gemäß Anspruch 10, **dadurch gekennzeichnet, dass** das gegenüber Strahlung abschirmende Material (50) durch einen Schlitz (51) unterbrochen ist, der sich in Längsrichtung des länglichen Sondenkörpers erstreckt.

12. Vorrichtung gemäß Anspruch 11, **dadurch gekennzeichnet, dass** der Unterbrechungsschlitz ein n · ¼- Kreissegment, wobei n gleich 1, 2 oder 3 ist, aufweist.

13. Vorrichtung gemäß einem der Ansprüche 1 bis 12, **dadurch gekennzeichnet, dass** die Energie emittierende Quelle eine Ir-192-Quelle mit hoher Dosisrate ist.

14. Vorrichtung gemäß einem der Ansprüche 1 bis 12, **dadurch gekennzeichnet, dass** die Energie emittierende Quelle eine Ir-192-Quelle mit gepulster Dosisrate ist.

15. Vorrichtung gemäß einem der Ansprüche 1 bis 12, **dadurch gekennzeichnet, dass** die Energie emittierende Quelle eine Miniatur-Röntgenstrahlquelle ist.

16. Vorrichtung gemäß einem der Ansprüche 1 bis 12, **dadurch gekennzeichnet, dass** die Energie emittierende Quelle eine Radiowellen emittierende Quelle ist.

17. Vorrichtung gemäß einem der vorangehenden Ansprüche, **dadurch gekennzeichnet, dass** die Dosisverteilung, die mit der Vielzahl von Energie emittierenden Quelle, die in der Vielzahl von hohlen Führungskanälen angeordnet sind, die gleichmässige Form der Aussenseite der Ballonwand des ersten aufblasbaren Ballonsystems im umgebenden Gewebe folgt.

18. Vorrichtung gemäß einem der vorangehenden Ansprüche, **dadurch gekennzeichnet, dass** die Vorrichtung einen Katheter mit offener Drainage zum Einsetzen in den hohlen, länglichen Sondenkörper zur Drainage von Körperflüssigkeiten aus dem Cavum nach der angewandten Strahlenbehandlung umfasst.

## Revendications

1. Dispositif (30) pour un traitement par rayonnement d'un tissu prolifératif entourant une cavité (2) dans un corps d'animal (1) comprenant :
- au moins un système de ballonnet gonflable (33) ayant une paroi de ballonnet pour la mise en place dans ladite cavité (2) ;
- une sonde de support (31) ayant une surface circonférentielle externe et un corps creux allongé avec une extrémité distale reliée audit système de ballonnet gonflable et une extrémité proximale restant à l'extérieur de ladite cavité (2) ;
- des moyens de gonflement pour gonfler et dégonfler ledit système de ballonnet (33) ;
- des moyens d'apport de rayonnement pour la mise en place d'au moins une source émettant de l'énergie (22) dans ladite cavité (2) pour réaliser ledit traitement par rayonnement, où lesdits moyens d'apport de rayonnement comprennent au moins un canal de guidage creux pour guider ladite au moins une source émettant de l'énergie à l'intérieur de ladite cavité,
**caractérisé en ce que**
ledit système de ballonnet gonflable (33) comprend deux ou plusieurs chambres de ballonnet (34a-34f) disposées autour de l'extrémité distale de ladite sonde de support (31), qui, quand elles sont gonflées, sont capables de se conformer totalement à la surface interne de la cavité (2) et où ledit au moins un canal de guidage (35a-35f) est positionné entre et à l'extérieur de chambres de ballonnet (34a-34f) adjacentes sur la surface circonférentielle externe de la sonde de support.

2. Dispositif selon la revendication 1 **caractérisé en ce que** lesdites chambres de ballonnet sont formées en reliant localement (38a-38f) ladite paroi de ballonnet au corps de sonde allongé (31), en formant et créant ainsi un certain nombre de plis de paroi de ballonnet.

3. Dispositif selon la revendication 2 **caractérisé en ce que** ledit nombre de plis de paroi de ballonnet est égal au nombre de canaux de guidage.

4. Dispositif selon la revendication 2 ou 3 **caractérisé en ce que** lesdites liaisons (38a-38f) entre ladite paroi de ballonnet et ledit corps allongé s'étendent dans la direction longitudinale du corps de sonde allongé (31).

5. Dispositif selon la revendication 4 **caractérisé en ce que** lesdites liaisons sont orientées parallèlement à l'orientation des canaux de guidage sur ledit corps de sonde allongé.

6. Dispositif selon la revendication 4 ou 5 **caractérisé en ce que** lesdites liaisons entre ladite paroi de ballonnet et ledit corps de sonde allongé sont établies par collage.

7. Dispositif selon la revendication 6 ou 7 **caractérisé en ce que** lesdites liaisons entre ladite paroi de ballonnet et ledit corps de sonde allongé sont établies par soudage.

8. Dispositif selon l'une quelconque des revendications précédentes **caractérisé en ce que** ledit système de ballonnet présente une surface de paroi de ballonnet plus grande que la surface du tissu proliféractif entourant ladite cavité.

9. Dispositif selon l'une quelconque des revendications précédentes **caractérisé en ce que** ledit système de ballonnet gonflable est formé à partir d'une feuille flexible, par exemple en polyuréthane.

10. Dispositif selon l'une quelconque des revendications précédentes **caractérisé en ce qu'**un matériau formant écran au rayonnement (50) est positionné à l'intérieur dudit corps de sonde allongé creux (31) et couvrant au moins partiellement la surface interne dudit corps de sonde allongé creux.

11. Dispositif selon la revendication 10 **caractérisé en ce que** ledit matériau formant écran au rayonnement (50) est interrompu au moyen d'une fente (51) s'étendant dans la direction longitudinale du corps de sonde allongé.

12. Dispositif selon la revendication 11 **caractérisé en ce que** ladite fente d'interruption présente un segment de n.¼ de cercle, n étant 1, 2 ou 3.

13. Dispositif selon l'une quelconque des revendications 1-12 **caractérisé en ce que** la source émettant de l'énergie est une source de Ir-192 à haut débit de dose.

14. Dispositif selon l'une quelconque des revendications 1-12 **caractérisé en ce que** la source émettant de l'énergie est une source de Ir-192 à débit de dose pulsé.

15. Dispositif selon l'une quelconque des revendications 1-12 **caractérisé en ce que** la source émettant de l'énergie est une source de rayons X miniature.

16. Dispositif selon l'une quelconque des revendications 1-12 **caractérisé en ce que** la source émettant de l'énergie est une source émettant des ondes radio.

17. Dispositif selon l'une quelconque des revendications précédentes **caractérisé en ce que** la distribution de dose produite avec ladite pluralité de sources émettant de l'énergie positionnées dans ladite pluralité de canaux de guidage creux suivre la forme conforme de l'extérieur de la paroi de ballonet du premier système de ballonet gonflable, qui est entouré par le tissu.

18. Dispositif selon l'une quelconque des revendications précédentes **caractérisé en ce que** le dispositif comprend un cathéter de drainage ouvert pour l'insertion dans le corps de sonde allongé creux pour drainer les liquides corporels à l'extérieur de la cavité après le traitement par rayonnement appliqué.
